# EUROPÄISCHE PATENTANMELDUNG

(11) **EP 1 400 249 A1**
(43) Veröffentlichungstag der Anmeldung: **24.03.2004**
(21) Anmeldenummer: 03450211.2
(22) Anmeldetag: 18.09.2003
(51) Int. Cl.: A61K 47/18, A61K 31/196

(54) **Pharmazeutische Zubereitung von Diclofenac-Natrium**

(30) Priorität: 19.09.2002 AT 14072002
(71) Anmelder: Mayrhofer Pharmazeutika Gesellschaft m.b.h., 4021 Linz (AT)
(72) Erfinder: Pfanner, Herbert, 4540 Bad Hall (AT)
(74) Vertreter: Hübscher, Helmut, Dipl.-Ing.

(57) **Zusammenfassung**

Es wird eine Verpackungseinheit für eine Infusionslösung aus Diclofenac-Natrium in Form einer in einem Behälter abgefüllten, sterilisierten, gepufferten, wäßrigen Infusionslösung beschrieben. Um eine stabile Infusionslösung zu erhalten, wird vorgeschlagen, daß die wäßrige Infusionslösung neben Diclofenac-Natrium als pharmazeutisch wirksame Monosubstanz in an sich bekannter Weise eine physiologisch unbedenkliche Aminosäure und eine physiologisch unbedenkliche Base als Puffersubstanzen enthält, in einem Behälter aus Kunststoff verpackt und durch Wärmeeinwirkung sterilisiert ist.

## Beschreibung

Die Erfindung bezieht sich auf eine Verpackungseinheit für eine Infusionslösung aus Diclofenac-Natrium in Form einer in einem Behälter abgefüllten, sterilisierten, gepufferten, wäßrigen Infusionslösung.

Wegen der begrenzten, mit abnehmendem pH-Wert abnehmenden Wasserlöslichkeit von Diclofenac-Natrium, einem Natriumsalz der [2-(2,6-Dichloranilino)-phenyl]essigsäure, ist es schwierig, stabile wäßrige Infusionslösungen aus Diclofenac-Natrium als pharmazeutisch wirksame Monosubstanz herzustellen, weil bei einem physiologisch verträglichen pH-Wert vorzugsweise zwischen 7,4 und 8,0 beim Einsatz üblicher Pufferlösungen, beispielsweise auf der Basis von Phosphaten oder Citraten, nach einem Sterilisieren der Infusionslösung durch Wärmeeinwirkung ein feinkristalliner Niederschlag auftritt.

Um den pH-Wert einer Injektionslösung auf der Basis von Diclofenac-Natrium unter 8,5 absenken zu können, wurde bereits vorgeschlagen (EP236 855 A1), eine physiologisch unbedenkliche Aminosäure, eine matrixbildende Substanz und Diclofenac-Natrium in einer wäßrigen Lösung einer physiologisch unbedenklichen Base zu lösen, wobei die Base so bemessen wird, daß die entstehende Lösung einen pH-Wert zwischen 8 und 10 aufweist. Die erhaltene Lösung wird nach einem Sterilfiltrieren lyophilisiert, um das Lyophilisat in einer sauren Lösung aufzulösen, so daß der pH-Wert der erhaltenen Injektionslösung auf einen Wert zwischen 7,0 und 8,5 eingestellt werden kann. Zum Lösen des Lyophilisats wird vorzugsweise eine Lösung von Salzen der B-Vitamingruppe verwendet. Nachteilig bei dieser bekannten Injektionslösung ist neben dem vergleichsweise großen Herstellungsaufwand, daß zusätzlich eine matrixbildende Substanz, z. B. Dextran, eingesetzt werden muß, damit der Lyophilisatkuchen eine für seine spätere Lösung erforderliche lockere Struktur erhält. Außerdem ist die Sterilisation auf ein Sterilfiltern beschränkt.

Der Erfindung liegt somit die Aufgabe zugrunde, eine Diclofenac-Natrium als pharmazeutisch wirksame Monosubstanz enthaltende wäßrige Infusionslösung anzugeben, die unter Wärmeeinwirkung sterilisiert werden kann und trotz der Wärmesterilisation eine hohe chemische und physikalische Stabilität als Voraussetzung für eine gute Lagerfähigkeit aufweist.

Ausgehend von einer Verpackungseinheit für eine Infusionslösung aus Diclofenac-Natrium der eingangs geschilderten Art löst die Erfindung die gestellte Aufgabe dadurch, daß die wäßrige Infusionslösung neben Diclofenac-Natrium als pharmazeutisch wirksame Monosubstanz in an sich bekannter Weise eine physiologisch unbedenkliche Aminosäure und eine physiologisch unbedenkliche Base als Puffersubstanzen enthält, in einem Behälter aus Kunststoff verpackt und durch Wärmeeinwirkung sterilisiert ist.

Durch den an sich bekannten Einsatz einer physiologisch unbedenklichen Aminosäure, beispielsweise Glycin, sowie einer physiologisch unbedenklichen Base, vorzugsweise Natronlauge, als Puffersubstanzen kann in überraschender Weise eine chemisch und physikalisch stabile Infusionslösung mit einem pH-Wert zwischen 7,4 und 8,0 erhalten werden, die sich zum Sterilisieren unter Wärmeeinwirkung eignet, ohne nach der Wärmesterilisation einen feinkristallinen Niederschlag befürchten zu müssen. Dies ist allerdings nur möglich, wenn die Infusionslösung in Kunststoffbehälter abgefüllt wird. Bei der Verwendung von Glasflaschen tritt trotz der nicht üblichen Pufferlösung ein solcher Niederschlag auf. Obwohl sich unterschiedliche Kunststoffe für die Behälter zum Abfüllen der Infusionslösung eignen, ergibt sich mit einem Behälter aus Polypropylen für die Infusionslösung eine besonders vorteilhafte, langlebige Verpackungseinheit.

Zum Herstellen einer erfindungsgemäß gepufferten Infusionslösung aus Diclofenac-Natrium als pharmazeutisch wirksame Monosubstanz werden eine physiologisch unbedenkliche Aminosäure sowie eine physiologisch unbedenkliche Base als Puffersubstanzen sowie das Diclofenac-Natrium in Wasser für Injektionszwecke gelöst, bevor die erhaltene Infusionslösung vorzugsweise nach einem Filtrieren über einen Sterilfilter in Kunststoffbehälter abgefüllt und anschließend unter Wärmeeinwirkung sterilisiert wird. Die abschließende Wärmesterilisation stellt in einfacher Weise eine sterile Infusionslösung sicher, und zwar unter der Voraussetzung der Anwendung üblicher pharmazeutischer Herstellverfahren. Trotz dieser vorteilhaften Wärmesterilisation kann aufgrund der gewählten Pufferlösung in Verbindung mit der Abfüllung der Infusionslösung in Kunststoffbehälter ein stabiles Verhalten sowohl in chemischer als auch in physikalischer Hinsicht über lange Zeiträume gewährleistet werden.

Selbstverständlich können der Infusionslösung übliche Zusatzstoffe zugemengt werden, um beispielsweise einen im Zusammenhang mit der Anwendung einzuhaltenden osmotischen Druck durch die Beigabe entsprechender Kochsalzmengen einstellen zu können.

### Ausführungsbeispiel

Es wurden in 300 l Wasser für Injektionszwecke mit einer Temperatur von 40°C nacheinander 175g Natronlaugeplätzchen, 150g Diclofenac-Natrium, 3500g Glycin sowie 2650g Natriumchlorid zur Einstellung der Osmolarität gelöst. Die klare Infusionslösung wurde mit Wasser für Injektionszwecke auf 500 l aufgefüllt. Danach wurde die Lösung über ein Sterilfilter mit einer Porengröße von 0,2µm filtriert, bevor die filtrierte Infusionslösung in leere, gewaschene Kunststoffinfusionsflaschen aus Polypropylen mit einem Inhalt von 250 ml unter entsprechenden Bedingungen abgefüllt wurde. Die Kunststoffinfusionsflaschen wurden mit einem Stopfen verschlossen und verbördelt. Anschließend erfolgte eine Sterilisation bei 121°C während 15 Minuten. Es ergaben sich lagerfähige Verpackungseinheiten von 250 ml Infusionslösung mit 0,03 % Diclofenac-Natrium als pharmazeutisch wirksame Monosubstanz.

## Patentansprüche

1. Verpackungseinheit für eine Infusionslösung aus Diclofenac-Natrium in Form einer in einem Behälter abgefüllten, sterilisierten, gepufferten, wäßrigen Infusionslösung, **dadurch gekennzeichnet, daß** die wäßrige Infusionslösung neben Diclofenac-Natrium als pharmazeutisch wirksame Monosubstanz in an sich bekannter Weise eine physiologisch unbedenkliche Aminosäure und eine physiologisch unbedenkliche Base als Puffersubstanzen enthält, in einem Behälter aus Kunststoff verpackt und durch Wärmeeinwirkung sterilisiert ist.

2. Verpackungseinheit nach Anspruch 1, **dadurch gekennzeichnet, daß** der Kunststoffbehälter aus Polypropylen besteht

3. Verfahren zum Herstellen einer wäßrigen, sterilisierten, gepufferten Infusionslösung aus Diclofenac-Natrium als pharmazeutisch wirksame Monosubstanz, **dadurch gekennzeichnet, daß** das Diclofenac-Natrium und eine physiologisch unbedenkliche Aminosäure sowie eine physiologisch unbedenkliche Base als Puffersubstanzen gegebenenfalls mit Zusatzstoffen in Wasser für Injektionszwecke gelöst werden und daß die so erhaltene Infusionslösung in Kunststoffbehälter abgefüllt und anschließend unter Wärmeeinwirkung sterilisiert wird.
